# EUROPEAN PATENT SPECIFICATION

(11) **EP 2 710 122 B1**
(45) Date of publication and mention of the grant of the patent: **31.03.2021**
(21) Application number: 12723442.5
(22) Date of filing: 18.05.2012
(51) Int. Cl.: C12N 5/0783, A61K 39/00, A61P 37/00, A61K 35/14

(54) **CELL POPULATIONS HAVING IMMUNOREGULATORY ACTIVITY, METHODS FOR THE PREPARATION AND USES THEREOF**
ZELLPOPULATIONEN MIT IMMUNREGULATORISCHER AKTIVITÄT, VERFAHREN ZU IHRER HERSTELLUNG UND VERWENDUNG
POPULATIONS CELLULAIRES AYANT UNE ACTIVITÉ IMMUNORÉGULATRICE, LEURS PROCÉDÉS DE PRÉPARATION ET LEURS UTILISATIONS

(30) Priority: 19.05.2011 EP 11166808
(43) Date of publication of application: 26.03.2014
(73) Proprietor: TiGenix, S.A.U., 28760 Tres Cantos - Madrid (ES)
(72) Inventor: DE LA ROSA, Olga, E-28760 Tres Cantos (ES)
(74) Representative: Lasar, Andrea Gisela
(86) International application number: PCT/EP2012/059313
(87) International publication number: WO 2012/156522

(56) References cited:
- EP-A1- 2 050 814
- WO-A1-2011/048222
- WO-A2-2007/039150
- CORREALE J & VILLA A: "Role of CD8+ CD25+ Foxp3+ regulatory T cells in multiple sclerosis", ANNALS OF NEUROLOGY, vol. 67, no. 5, May 2010 (2010-05), pages 625-638, XP55034925, ISSN: 0364-5134, DOI: 10.1002/ana.21944 [retrieved on 2009-12-08]
- BEN-AMI E ET AL: "Mesenchymal stem cells as an immunomodulatory therapeutic strategy for autoimmune diseases", AUTOIMMUNITY REVIEWS, vol. 10, no. 7, May 2011 (2011-05), pages 410-415, XP55034927, ISSN: 1568-9972, DOI: 10.1016/j.autrev.2011.01.005 [retrieved on 2011-01-20]
- YANEZ R ET AL: "Adipose tissue-derived mesenchymal stem cells have in vivo immunosuppressive properties applicable for the control of the graft-versus-host disease", STEM CELLS, vol. 24, no. 11, November 2006 (2006-11), pages 2582-2591, XP002620770, ISSN: 1066-5099, DOI: 10.1634/STEMCELLS.2006-0228 [retrieved on 2006-07-27]
- CLARK R A & KUPPER T S: "IL-15 and dermal fibroblasts induce proliferation of natural regulatory T cells isolated from human skin", BLOOD, vol. 109, no. 1, 1 January 2007 (2007-01-01), pages 194-202, XP55034928, ISSN: 0006-4971, DOI: 10.1182/blood-2006-02-002873
- DELAROSA O ET AL: "Requirement of IFN-gamma-mediated indoleamine 2,3-dioxygenase expression in the modulation of lymphocyte proliferation by human adipose-derived stem cells", TISSUE ENGINEERING, vol. 15, no. 10, 7 October 2009 (2009-10-07), pages 2795-2806, XP002563369, ISSN: 1076-3279, DOI: 10.1089/TEN.TEA.2008.0630 [retrieved on 2009-04-03] cited in the application
- SEDDIKI N ET AL: "Expression of interleukin (IL)-2 and IL-7 receptors discriminates between human regulatory and activated T cells", JOURNAL OF EXPERIMENTAL MEDICINE, vol. 203, no. 7, 10 July 2006 (2006-07-10) , pages 1693-1700, XP55034957, ISSN: 0022-1007, DOI: 10.1084/jem.20060468

### SEQUENCE LISTING

<110> TiGenix SA de la Rosa, Olga
<120> CELL POPULATIONS HAVING IMMUNOREGULATORY ACTIVITY, METHODS FOR THE PREPARATION AND USES THEREOF
<130> CLX-P1414PCT
<160> 8
<170> PatentIn version 3.5
<210> 1
   <211> 20
   <212> PRT
   <213> Homo sapiens
<400> 1 Leu Pro Arg His 20
<210> 2
   <211> 17
   <212> PRT
   <213> Homo sapiens
<400> 2 **Pro**
<210> 3
   <211> 19
   <212> PRT
   <213> Homo sapiens
<400> 3 Tyr Gly Gly
<210> 4
   <211> 25
   <212> PRT
   <213> Homo sapiens
<400> 4
   Ala Gln Gly Thr Leu Ser Lys Ile Phe Lys Leu Gly Gly Arg Asp Ser
   1               5                   10                  15
   Arg Ser Gly Ser Pro Met Ala Arg Arg
               20                  25
<210> 5
   <211> 20
   <212> PRT
   <213> Homo sapiens
<400> 5 Gly Asp Glu Val 20
<210> 6
   <211> 21
   <212> PRT
   <213> Homo sapiens
<400> 6 Tyr Arg Asn Gly Lys 20
<210> 7
   <211> 16
   <212> PRT
   <213> Homo sapiens
<400> 7
   His Cys Leu Gly Lys Trp Leu Gly His Pro Asp Lys Phe Val Gly Ile
   1               5                   10                  15
<210> 8
   <211> 13
   <212> PRT
   <213> Influenza virus
<400> 8
   Pro Lys Tyr Val Lys Gln Asn Thr Leu Lys Leu Ala Thr
   1               5                   10

## Description

### FIELD OF THE INVENTION

The present invention relates to methods for providing immunomodulatory cells. This disclosure also provides therapeutic uses of the cells for the immune modulation of mammals in need thereof.

### BACKGROUND OF THE INVENTION

Regulatory T-cells: All immune responses are controlled by T cells. Self-reactive cells with the potential to elicit autoimmune responses comprise a part of the normal T cell repertoire, but in the healthy state, their activation is prevented by suppressor cells. Although T suppressor cells were originally described in the 1970s, significant progress in characterizing T-cell subsets has been made only recently, when they have been renamed as regulatory T cells (Treg cells).

There are different CD4+, CD8+, natural killer cell, and gamma and delta T cell subsets with regulatory (suppressor) activity. Two major types of Treg cells have been characterized in the CD4+ population, namely the naturally-occurring, thymus-generated Treg cells, and the peripherally-induced, IL-10 or TGF-beta secreting T-reg cells (TrI cells). The CD4+CD25+, Foxp3 -expressing, naturally-occurring T-reg cells generated in thymus, migrate and are maintained in the periphery.

Methods for the *in-vitro* preparation of Treg cells (for their use in the treatment of immune and inflammatory disorders) are known in the art. For example International Patent Application WO2011/048222 provides a method for the preparation of Treg cells by contacting mesenchymal stem cells with peripheral blood leukocytes.

Adipose-derived stem cell induction of Tregs: Human adipose-derived mesenchymal stem cells (hASC) are a source of multipotent adult stem cells capable of differentiation into poorly immunogenic mesenchymal-type cells expressing low levels of HLA class I, but lacking HLA class II, CD40, CD80 or CD86 molecules. Furthermore, expanded hASC have been reported to inhibit activation, proliferation and function of immune cells by both cell contact-dependent mechanisms and soluble factors secreted in response to cytokines released by activated immune cells. Experimental models for Rheumatoid Arthritis (RA) and Crohn's disease are the object of studies as models of autoimmune/inflammatory diseases, potential targets for hASC treatment. Data developed both in Collagen Induced Arthritis (CIA) and Inflammatory Bowel Disease (IBD) mice, reported that infusion of hASCs significantly reduced the incidence and severity of both diseases. It was demonstrated that hASC treatment had a protective effect when a subsequent induction of the disease peak was provoked. This clinical reduction of severity was accompanied by local and systemic anti-inflammatory effect mediated by the downregulation of the Th1 response. These data were accompanied by the fact that hASC were able to migrate to the lymphoid organs during a small period to further disappear. Further analysis demonstrated *de novo* generation of antigen-specific CD4+CD25+FoxP3+ regulatory T cells (Treg) with the capacity to suppress self-reactive T effector responses occurring after the treatment. The *ex vivo* studies performed in RA patients indicated that hASCs exert profound suppressive responses on collagen-reactive T cells by various mechanisms. One of them was the generation of collagen specific Treg cells that inhibit the proliferation of autoreactive T cells. It appears that hASC exert their immunosuppressive activity by the selective induction of CD4+CD25brightFOXP3+ Tregs with suppressor capacity over self-PBMCs in a dose dependent manner.

Multiple sclerosis: Multiple Sclerosis (MS) is an autoimmune condition in which the immune system attacks the central nervous system (CNS) through a chronic inflammatory and demyelinising process of the CNS. Induction of remission in MS has been associated with stimulation of CD4+CD25+FOXP3+ Tregs playing an important role in the prevention of autoimmunity. Numerous studies have reported numeric or functional deficiencies of Treg in various human autoimmune diseases including inflammatory and demyelinating disorders of the CNS. Studies of MS patients further support the hypothesis that restoration of Treg function is a promising therapeutic approach in humans. In fact, suboptimal suppressive capacity of Tregs has been observed in patients with relapsing-remitting MS. For example, patients responding to the clinically used immune modulator drug glatiramer acetate have been reported to have increased levels of CD4+CD25+FoxP3+ Treg cells in peripheral blood and cerebral spinal fluid. Interferon beta, another clinically used drug for MS induces a renormalization of Treg activity after initiation of therapy through stimulation of *de novo* generation of Tregs. In the animal model of MS, experimental allergic encephalomyelitis (EAE), disease progression is exacerbated by Treg depletion, and natural protection against disease in certain models of EAE is associated with antigen-specific Treg. These data suggest that the immune dysfunction in MS patients may be intrinsic to Tregs rather than a result of effector T cell resistance to suppression.

EP2050814 describes compositions comprising Trl cells directed to a multiple sclerosis associated antigens and methods for treating multiple sclerosis. Correale & Villa (2010) Ann. Neurol. 67(5):625-638 discloses an investigation into the role of CD8+ CD25+ FoxP3+ regulatory T cells during the course of multiple sclerosis. Ben-Ami et al. (2011) Autoimmun. Rev. 10(7):410-415 describes a review of the immunomodulatory capabilities of mesenchymal stem cells (MSCs). WO2007/039150 describes a population of connective tissue derived cells that respond to interferon-gamma by expressing indolamine-2,3-dioxygenase (IDO) for use in treating one or more symptoms associated with disorders in which modulation of a subject's immune system is beneficial, such as autoimmune diseases. Yañez et al. (2006) Stem Cells 24(1):2582-2591 discloses an investigation to show that adipose tissue-derived mesenchymal stem tells have immunosuppressive properties applicable for the control of Graft-versus-Host Disease. Clark & Kupper (2007) Blood 109(1):194-202 describes the isolation and expansion of a Treg population resident in normal human skin.

### SUMMARY OF THE INVENTION

In one aspect, the invention relates to a method for the preparation, expansion and/or generation of antigen-specific immunomodulatory cells which comprises *ex-vivo* contacting an isolated regulatory T cell population with a mesenchymal stem cell (MSC) population in the presence of one or more multiple sclerosis-associated antigens selected from the group consisting of: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6), PLP 139-154 (SEQ ID NO :7).

In another aspect, the invention relates to a method for preparing a pharmaceutical composition for use in treating multiple sclerosis, comprising the steps of:
i) providing a PBL population;
ii) contacting said PBLs with a cell population comprising mesenchymal stem cells (MSC) in the presence of one or more multiple sclerosis-associated antigens selected from the group consisting of: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6), PLP 139-154 (SEQ ID NO: 7);
iii) isolating the immunomodulatory cell population;
iv) determining the expression of one or more markers selected from the group consisting of CD62-L, FOXP3 and CTLA4; and
v) selecting cells positive for at least 1, 2 or 3 of said markers.

### BRIEF DESCRIPTION OF THE FIGURES

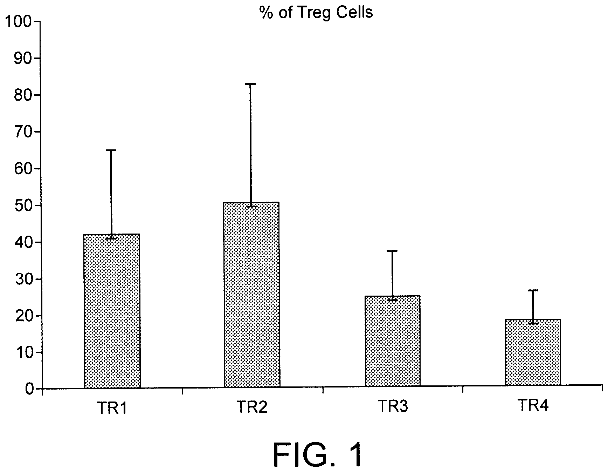

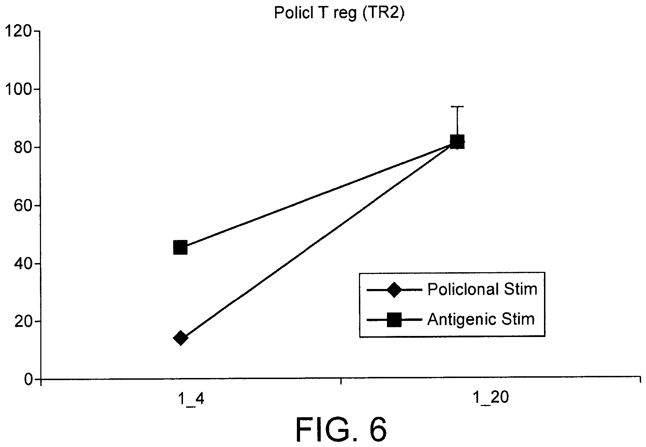

Figure 1 - Percentage of Treg cells generated under different conditions.
   TR1 represents co-cultures of PBLs and hASC with MS pooled peptides. TR2 represents co-cultures with hASC and no peptides. TR3 represents cultures performed in the absence of hASC and in the presence of pooled peptides. Tr4 represents cultures performed in the absence of hASC and pooled peptides. Graph indicates mean and standard deviation of three independent experiments.

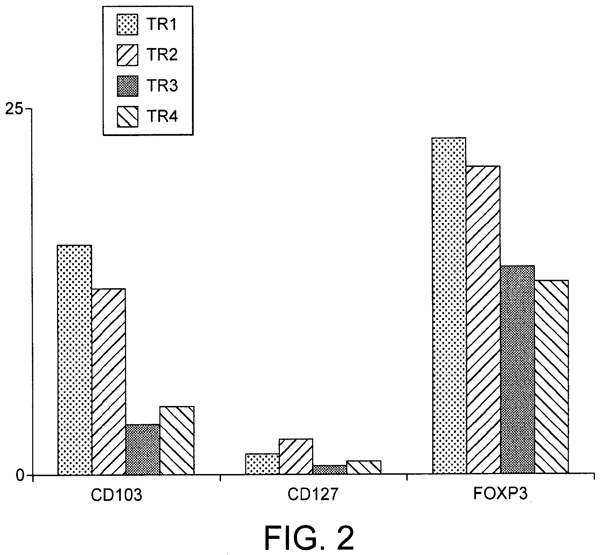

Figure 2 - Phenotype of Treg cells generated under different conditions.
   TR1 represents co-cultures of PBL with hASC and MS pooled peptides. TR2 represents co-cultures of PBL with hASC and no peptides. TR3 represents cultures performed in the absence of hASC and in the presence of pooled peptides. TR4 represents cultures performed in the absence of hASC and pooled peptides. Graph indicates mean of the percentage of expression of CD103, CD127 and intracellular stained FOXP-3 over the gated population of CD4+CD25bright (Treg) population. Three independent experiments were performed for each group.

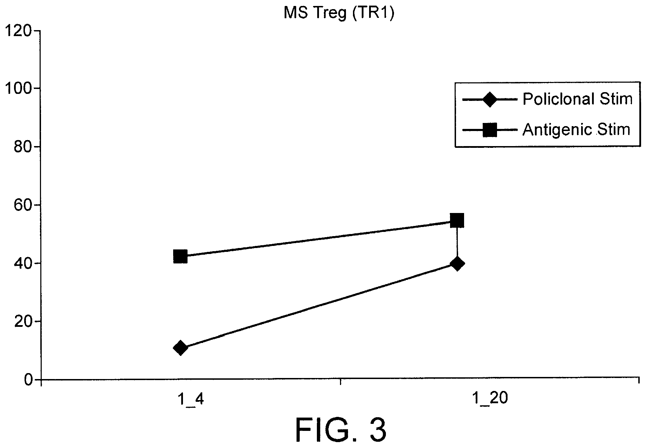

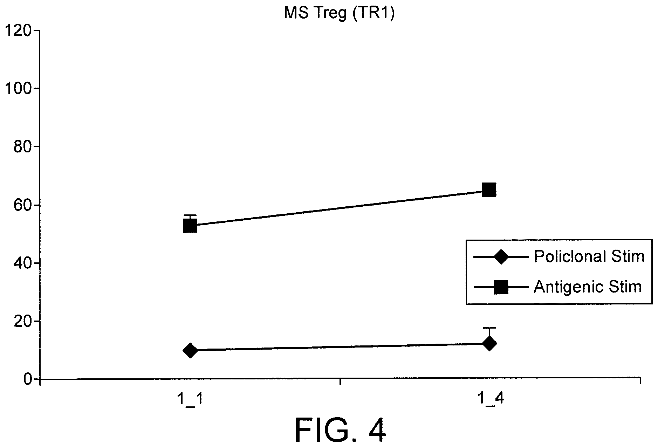

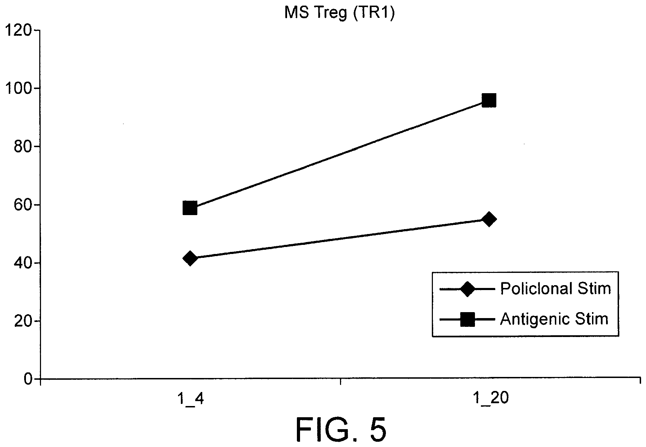

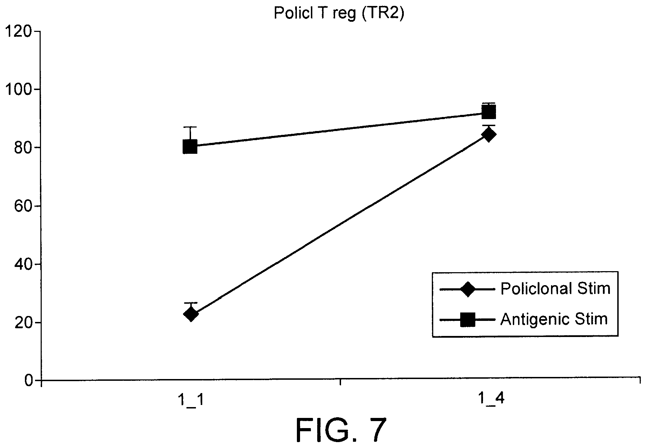

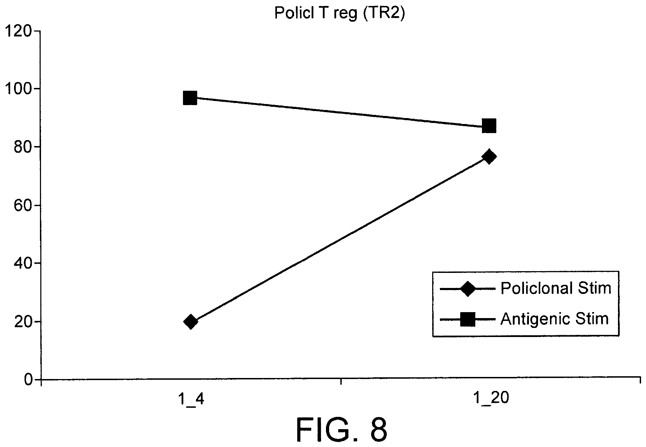

Figures 3-8 - Inhibition of the proliferation of polyclonal and antigen driven Treg cells.
   TR1 (Figures 3-5) represents co-cultures with hASC and MS pooled peptides. TR2 (Figures 6-8) represents co-cultures with hASC and no peptides. Upper row indicates the percentage of proliferation with antigen driven Treg cells using as stimulator pooled peptides or anti CD3/CD28 microbeads. Lower row indicate percentage of proliferation with polyclonal Treg cells using as stimulator the pooled peptide or the polyclonal stimulation. Experiments were performed at 1:1, 1:4 or 1:20 ratio (Treg: PBMC) depending on the number of cells isolated from each donor. Three independent experiments were performed for each group (TR1/TR2).

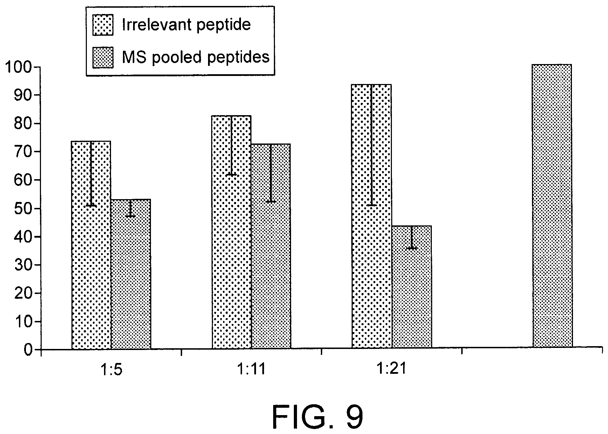

Figure 9 - Inhibition of the proliferation in antigen specific and non specific driven lymphocytes. Co-cultures with hASC and MS pooled peptides are represented. Maximum proliferation reached is 100% shown in the black right bar. Grey bars indicates the proliferation (percentage of proliferation referred to the maximum proliferation reached in the culture) of the different ratios of CD3 positive T cells in the presence of the irrelevant peptide Flu HA. Black bars indicates the proliferation (percentage of proliferation referred to the maximum proliferation reached in the culture) of the different ratios of CD3 positive T cells in the presence of the pooled MS peptides. Experiments were performed at 1:5, 1:11 or 1:21 ratio (Treg: PBMC). Mean and standard deviation of triplicates is shown in the graph. (Maximum proliferation reached is 100% shown in the black right bar).

### DETAILED DESCRIPTION OF THE INVENTION

The present invention provides methods for the preparation, expansion and/or generation of immunomodulatory cells having immunomodulatory properties. The immunomodulatory cells and uses thereof comprise further aspects of the disclosure.

### DEFINITIONS

In order to facilitate the understanding of the present description, the meaning of some terms and expressions in the context of the invention will be explained below. Further definitions will be included throughout the description as necessary.

The term "allogeneic" as used herein shall be taken to mean from different individuals of the same species. Two or more individuals are said to be allogeneic to one another when the genes at one or more loci are not identical.

The term "autologous" as used herein shall be taken to mean from the same individual.

The term "antigen presenting cells" (APC) refers to a cell population that displays surface foreign antigen complexed with major histocompatibility complex MHC. Although almost every cell in the body is capable of presenting antigens to T cells, the term "antigen presenting cells" (APC) is herein limited to those specialized cells that express surface MHC II (HLA DP, DQ, DR), and include both those cells in which this expression is induced (for example but not limited to B-cells and CD4 PHA blasts) and also those cells that are derived from the monocyte-macrophage lineage (for example, but not limited to, dendritic cells).

The term "isolated" applied to a cell population refers to a cell population, isolated from the human or animal body, which is substantially free of one or more cell populations that are associated with said cell population *in vivo* or *in vitro.*

The term "MHC" (major histocompatibility complex) refers to a subset of genes that encodes cell-surface antigen-presenting proteins. In humans, these genes are referred to as human leukocyte antigen (HLA) genes. Herein, the abbreviations MHC or HLA are used interchangeably. The term "subject" refers to an animal, preferably a mammal including a non- primate (e.g. a cow, pig, horse, cat, dog, rat, or mouse) and a primate (e.g. a monkey, or a human). In a preferred embodiment, the subject is a human.

The term "immunomodulatory" refers to the inhibition or reduction of one or more biological activities of the immune system this includes but is not limited to downregulation of immune response and inflammatory states as well as changes in cytokine profile, cytotoxic activity and antibody production.

The term "antigen specific" when used in the context of immunomodulatory cells refers to cells having the capacity of inhibition or reduction of one or more biological activities of the immune system (such as but not limited to T-cell proliferation) associated with or activated by a specific antigen or antigens, including both alloantigens and autoantigens.

The term "immunomodulatory" shall be taken to comprise "antigen specific immunomodulatory".

The terms "immunomodulatory agent", "immunomodulatory cell population", "immunomodulatory cell" or "immunomodulatory cells" as used herein shall be taken to mean agents, cell(s) or populations thereof that inhibit or reduce one or more biological activities (for example, but not limited to, the proliferation, differentiation, priming, effector function, production of cytokines or expression of antigens) of one or more immune cells (for example, but not limited to, T cells).

The term "T-cell" refers to cells of the immune system which are a subset of lymphocytes that express the T cell receptor (TCR). The term "regulatory T-cells" (also referred to herein as T-reg cells) refers to T cell subsets that actively suppress activation of the immune system and prevent pathological self-reactivity, i.e. an autoimmune disease. The term "regulatory T-cells" or "T-reg cells" shall be taken to include both naturally occurring T-cells (also known as CD4⁺CD25⁺FoxP3⁺ T-reg cells) and adaptive T-cells (also known as Tr1 cells or Th3 cells) which do not express the FoxP3 molecule.

In a particularly preferred embodiment of the present invention said immunomodulatory agents, cell(s) or populations thereof are regulatory T-cells, however in an alternative embodiment of the method they may be cells of other phenotypes that have been modified such that they are capable of performing the immunosuppressive functions of regulatory T-cells. For example, cells of other phenotypes may have previous to said modification lacked one or more of the following capabilities: suppression of a mixed lymphocyte reaction; suppression of a cytotoxic T cell response; inhibition of DC maturation; inhibition of T cell production of inflammatory cytokines.

As used herein, "negative" or "-" as used with respect to cell surface markers shall be taken to mean that mean that, in a cell population, less than 20%, 10%, preferably less than 9%, 8%, 7%, 6%, 5%, 4%, 3%, 2%, 1 % or none of the cells express said marker. Expression of cell surface markers may be determined for example by means of flow cytometry for a specific cell surface marker using conventional methods and apparatus (for example a Beckman Coulter Epics XL FACS system used with commercially available antibodies and standard protocols known in the art).

As used herein the term "mesenchymal stem cell" (also referred to herein as "MSC") shall be taken to mean a multipotent cell type originally derived from the mesenchyme.

As used herein, the expression "significant expression" or its equivalent terms "positive" and "+" when used in regard to a cell surface marker shall be taken to mean that, in a cell population, more than 20%, preferably more than, 30%, 40%, 50%, 60%, 70%, 80%, 90%, 95% or all of the cells of the cells express said marker.

Expression of cell surface markers may be determined for example by means of flow cytometry for a specific cell surface marker using conventional methods and apparatus (for example a Beckman Coulter Epics XL FACS system used with commercially available antibodies and standard protocols known in the art) that show a signal for a specific cell surface marker in flow cytometry above the background signal using conventional methods and apparatus (for example, a Beckman Coulter Epics XL FACS system used with commercially available antibodies and standard protocols known in the art). The background signal is defined as the signal intensity given by a non-specific antibody of the same isotype as the specific antibody used to detect each surface marker in conventional FACS analysis. For a marker to be considered positive the specific signal observed is stronger than 20%, preferably stronger than, 30%, 40%, 50%, 60%, 70%, 80%, 90%, 500%, 1000%, 5000%, 10000% or above, than the background signal intensity using conventional methods and apparatus (for example a Beckman Coulter Epics XL FACS system used with commercially available antibodies and standard protocols known in the art).

Furthermore, commercially available and known monoclonal antibodies against said cell-surface markers (e.g. cellular receptors and transmembrane proteins) can be used to identify relevant cells.

The term "connective tissue" refers to tissue derived from mesenchyme and includes several tissues which are characterized in that their cells are included within the extracellular matrix. Examples of connective tissues include but are not limited to, adipose and cartilaginous tissues.

The term "fibroblast" as used herein shall be taken to include fibroblast like synovial cells.

As used herein, the terms "treat", "treatment" and "treating" when used directly in reference to a patient or subject shall be taken to mean the amelioration of one or more symptoms associated with multiple sclerosis wherein said amelioration results from the administration of the immunomodulatory cells.

The term "combination therapy" refers to the use of the immunomodulatory cells or pharmaceutical compositions comprising thereof together with other active agents or treatment modalities, for the amelioration of one or more symptoms associated with a disorder including, but not limited to, an inflammatory disorder, an autoimmune disease or an immunologically mediated disease including rejection of transplanted organs and tissues. These other agents or treatments may include known drugs and therapies for the treatment of such disorders such as but not limited to corticosteroids and non-steroidal anti-inflammatory compounds.

The term "PBLs" shall be taken to mean peripheral blood leukocytes, lymphocytes or monocytes,

The immunomodulatory cells, or pharmaceutical compositions thereof may also be combined with corticosteroids, non-steroidal anti-inflammatory compounds, or other agents useful in treating inflammation. The combined use of the agents with these other therapies or treatment modalities may be concurrent, or given sequentially, that is, the two treatments may be divided up such that said immunomodulatory cells or a pharmaceutical composition comprising thereof may be given prior to or after the other therapy or treatment modality. The attending physician may decide on the appropriate sequence of administering the immunomodulatory cells, or a pharmaceutical composition comprising thereof, in combination with other agents, therapies or treatment modalities.

### DETAILED DESCRIPTION

Disclosed herein are isolated immunomodulatory cells, populations and compositions thereof. The immunomodulatory cells have surprising efficacy in the immunomodulation of multiple sclerosis.

In one embodiment said immunomodulatory cells are regulatory T-cells, in a particularly preferred embodiment said immunomodulatory cells are Foxp3+CD4+CD25+ T-reg and/or IL-10/TGFb-producing regulatory Tr1 cells. The isolated immunomodulatory cells are preferably characterized in that they express (i.e. are positive for) at least one, two, three, four, five of or preferably all of the cell surface markers CD62-L, FOXP3, CTLA4, CD104 and GITR. Preferably, the MSC are characterised in that they have significant expression levels of at least one, two, three, four, of and preferably all of said cell surface markers (CD62-L, FOXP3, CTLA4, CD104 and GITR).

The isolated immunomodulatory cells are more preferably characterized in that they express (i.e. are positive for) at least one, two, or preferably all of the cell surface markers CD62-L, FOXP3 and CTLA4. Preferably, the MSC are characterised in that they have significant expression levels of at least one, two, or preferably all of said cell surface markers (CD62-L, FOXP3 and CTLA4). It is further preferred that they do not express (i.e. are negative for) CD127.

It is preferred that the isolated immunomodulatory cells are *ex-vivo* prepared, expanded or generated. The immunomodulatory cells are antigen specific for (or preferentially modulate immune response activated by) one or more multiple sclerosis-associated antigens selected from: Myelin Basic Protein peptides, Myelin Oligodendrocyte Glycoproteins and Proteolipid Proteins and fragments, variants and mixtures thereof, represented by SEQ ID NOs: 1-7.

The antigen specific immunomodulatory cells have been demonstrated as having improved efficacy in the modulation of immune response in an *in vitro* model of multiple sclerosis. The immunomodulatory cells are *ex-vivo* generated by exposure to one or more multiple sclerosis-associated antigens selected from the group consisting of: SEQ ID NOs: 1-7.

Also disclosed are populations of the immunomodulatory cells comprising essentially of said immunomodulatory cells, or alternatively at least 80%, 85%, 90%, 95% by cell number of said immunomodulatory cells.

In one aspect, the present invention relates to methods for the preparation, expansion and/or generation of immunomodulatory cells. In one embodiment said immunomodulatory cells are regulatory T-cells, in a particularly preferred embodiment said immunomodulatory cells are Foxp3+CD4+CD25+ T-reg and/or IL-10/TGFb-producing regulatory Tr1 cells. Said method comprises contacting a cell population comprising of MSC with PBLs in the presence of one or more multiple sclerosis-associated antigens selected from SEQ ID NOs: 1-7.

It is particularly preferred that said immunomodulatory cells express the cell surface markers CD62-L, FOXP3 and CTLA4. It is further preferred that they do not express, i.e. are negative for, CD127. Accordingly in an alternative embodiment the present invention provides pharmaceutical composition for use in treating multiple sclerosis comprising the steps of:
i) providing a PBL population;
ii) contacting said PBLs with a cell population comprising of MSC in the presence of one or more multiple sclerosis-associated antigens selected from the group consisting of: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6), PLP 139-154 (SEQ ID NO: 7);
iii) isolating the immunomodulatory cell population;
iv) determining the expression of one or more markers selected from the group consisting of CD62-L, FOXP3 and CTLA4; and
v) selecting cells positive for at least 1, 2 or 3 of said markers.

The term "MSC" and/or "fibroblast cell population" shall be used to mean any of: a plurality of cells comprising essentially of mesenchymal stem cells; a plurality of cells comprising essentially of fibroblasts; a plurality of cells comprising essentially of mesenchymal stem cells and fibroblasts. It is preferred that the ratio of number of cells in said MSC and/or fibroblast cell population to isolated regulatory T-cells is between 1:1 and 1:150 respectively. It is further preferred that the ratio of number of cells in said MSC and/or fibroblast cells to PBLs is between 1:30 and 1:5. Accordingly, in one embodiment this may be about 1 MSC to every 25 PBLs, or 1 MSC to every 10 PBLs.

In said method for preparing, expanding and/or generating immunoregulatory cells, MSC are cultured *in vitro* with PBLs. The culture period is preferably between 2 hours and 21 days, and is more preferably between 5 and 17 days. In a further embodiment said culture is carried out for at least 2, 4, 5, or 6 or more days. It is particularly preferred that the culture period is about 15 days. This co-culturing will result in the production of immunomodulatory cells, providing an expanded population of said PBLs which can be used for treatment of a subject.

The method(s) of the invention are preferably performed in a temperature and carbon dioxide controlled environment, e.g. in an incubator. The method is preferably performed at about mammalian body temperature, accounting for regional variations, e.g. 37 degrees centigrade. It is also preferred that the method of the invention is carried out in an environment where carbon dioxide concentration is between 0% and 10% and more preferably between 1% and 5%.

### Preparation of PBLs.

With respect to the intended recipient of the immunomodulatory cells as prepared by the above described method of the present invention, the PBLs used in said method may be of either autologous or allogeneic origin. However it is preferred that they are of autologous origin (i.e. that they were obtained from the subject who subsequently receives the immunomodulatory cells or any treatment, medicament or pharmaceutical composition thereof). Methods for the isolation of peripheral blood leukocytes from whole blood are known in the art and include the use of Ficoll-Hypaque and/or red blood cell lysis procedures or commercially available means such as the LeucoPREP™ cell separation device (Becton Dickinson & Co.) and HISTOPAQUE™ (Sigma Diagnostics) solution.

### MSC.

The MSC used in the method of the present invention are preferably derived from connective tissue. In a preferred embodiment said MSC are derived from adipose tissue and in a further preferred embodiment from the stromal fraction of the adipose tissue. In an alternative embodiment, said MSC are obtained from chondrocytes of the hyaline cartilage. In a further embodiment, said MSC are obtained from skin. In another embodiment, said MSC are obtained from bone marrow.

The MSC can be obtained from any suitable source of connective tissue from any suitable animal, most preferably humans. It is preferred that said cells are obtained from non-pathological mammalian sources, preferably post-natal (e.g. rodent; primate). In a preferred embodiment, the MSC are obtained from a source of connective tissue, such as, but not limited to, the stromal fraction of adipose tissue, hyaline cartilage, bone marrow or skin. Most preferably said the MSC of the method are obtained from non-pathological, post-natal, human stromal adipose tissue.

With respect to the intended recipient of the immunomodulatory cells as prepared by the method of the present invention, the MSC used in said above described method may be of either allogeneic (donor) or autologous (subject) origin. In one embodiment of the method said MSC are of allogeneic origin.

The MSC used in the method of the present invention are preferably characterized in that (i) they do not express markers specific for APCs, (ii) they do not express IDO constitutively, (iii) they express IDO upon stimulation with IFN-gamma, and in the case of MSC (iv) they present the capacity to be differentiated into at least two cell lineages.

### MSC Phenotype Markers.

The MSC used in the method of the present invention are preferably negative for markers associated with APC phenotypes. Accordingly it is preferred that said MSC are negative for at least one, two, three, four or preferably all of the following markers CD 11b; CD 11c; CD1 14; CD45; HLAI1. Furthermore, the MSC are preferably negative for at least one, two, or preferably all of the following cell surface markers CD31; CD34; CD 133.

In a particular embodiment, the MSC as used in the present method are preferably characterised in that they express (i.e. are positive for) at least one, two, three, four, of or preferably all of the following cell surface markers CD9, CD44, CD54, CD90 and CD 105. Preferably, the MSC are characterised in that they have significant expression levels of at least one, two, three, four, of and preferably all of said cell surface markers (CD9, CD44, CD54, CD90 and CD 105).

Optionally, the MSC may also be negative for the cell surface marker CD 106 (VCAM-1). Examples of MSC suitable for use in the method of the present invention are described in the art, for example in International Patent Application WO2007/039150.

### Differentiation.

The MSC suitable for use in the method of the present invention may present the capacity to proliferate and be differentiated into at least two, more preferably three, four, five, six, seven or more cell lineages. Illustrative, non-limiting examples of cell lineages into which said MSC can be differentiated include osteocytes, adipocytes, chondrocytes, tenocytes, myocytes, cardiomyocytes, hematopoietic-supporting stromal cells, endothelial cells, neurons, astrocytes, and hepatocytes. MSC can proliferate and differentiate into cells of other lineages by conventional methods. Methods of identifying and subsequently isolating differentiated cells from their undifferentiated counterparts can be also carried out by methods well known in the art.

### MSC Cell Culture.

Said MSC are also capable of being expanded *ex vivo.* That is, after isolation, said MSC can be maintained and allowed to proliferate *ex vivo* in culture medium. Such medium is composed of, for example, Dulbecco's Modified Eagle's Medium (DMEM), with antibiotics (for example, 100units/ml Penicillin and 100µg/ml Streptomycin) or without antibiotics, and 2 mM glutamine, and supplemented with 2-20% fetal bovine serum (FBS). It is within the skill of one in the art to modify or modulate concentrations of media and/or media supplements as necessary for the cells used. Sera often contain cellular and non-cellular factors and components that are necessary for viability and expansion. Examples of sera include fetal bovine serum (FBS), bovine serum (BS), calf serum (CS), fetal calf serum (FCS), newborn calf serum (NCS), goat serum (GS), horse serum (HS), porcine serum, sheep serum, rabbit serum, rat serum (RS), etc. It is also within the scope of the invention that if said MSC are of human origin, the cell culture medium is supplemented with a human serum, preferably of autologous origin. It is understood that sera can be heat- inactivated at 55-65deg.C if deemed necessary to inactivate components of the complement cascade. Modulation of serum concentrations, withdrawal of serum from the culture medium can also be used to promote survival of one or more desired cell types. Preferably, said MSC will benefit from FBS concentrations of about 2% to about 25%. In another embodiment, the MSC can be expanded in a culture medium of definite composition, in which the serum is replaced by a combination of serum albumin, serum transferrin, selenium, and recombinant proteins including but not limited to insulin, platelet-derived growth factor (PDGF), and basic fibroblast growth factor (bFGF) as known in the art.

Many cell culture media already contain amino acids, however some require supplementation prior to culturing of cells. Such amino acids include, but are not limited to, L-alanine, L- arginine, L-aspartic acid, L-asparagine, L cysteine, L-cystine, L-glutamic acid, L-glutamine, L-glycine, and the like.

Antimicrobial agents are also typically used in cell culture to mitigate bacterial, mycoplasmal, and fungal contamination. Typically, antibiotics or anti-mycotic compounds used are mixtures of penicillin/streptomycin, but can also include, but are not limited to amphotericin (Fungizone®), ampicillin, gentamicin, bleomycin, hygromycin, kanamycin, mitomycin, etc.

Hormones can also be advantageously used in cell culture and include, but are not limited to, D-aldosterone, diethylstilbestrol (DES), dexamethasone, b-estradiol, hydrocortisone, insulin, prolactin, progesterone, somatostatin/human growth hormone (HGH), etc.

### Expanded Cells.

In one embodiment the MSC may have been expanded prior to use in the method of the present invention. Methods for cell expansion are known in the art.

### Irradiated Cells.

In one embodiment the MSC may have been irradiated prior to their use in the method of the present invention. Irradiation of cells reduces their proliferative capabilities and survival times.

The irradiation may be carried out using a suitable controlled source of ionizing radiation, such a gamma irradiator device. The irradiation conditions must be experimentally adjusted by a person skilled in the art to determine the required exposure time to impart a radiation dose that causes the long term growth arrest of the MSC. In one embodiment said radiation dose is within a range selected from the group consisting of 1-100 Gy; 5-85 Gy, 10-70 Gy, 12-60 Gy however it is particularly preferred that said radiation dose is within the range of 15-45 Gy.

### IFN-Gamma Stimulated Cells.

In one embodiment the MSC may be stimulated with interferon gamma prior to use in the method of the present invention. IFN-gamma treatment of MSC for the stimulation thereof is known in the art and may be carried out by a person skilled in the art.

### Mitomycin C Treated MSC.

In one embodiment the MSC may be treated with Mitomycin C prior to use in the method of the present invention. Mitomycin C treatment of MSC is known in the art and may be carried out by a person skilled in the art.

Furthermore, if desired, the MSC can be subjected to a plurality of the treatments selected from the group consisting of irradiation, IFN-gamma and Mitomycin C prior to use in the method of the present invention.

The maintenance conditions of said MSC can also contain cellular factors that allow cells to remain in an undifferentiated form. It is apparent to those skilled in the art that, prior to differentiation, supplements that inhibit cell differentiation must be removed from the culture medium. It is also apparent that not all cells will require these factors. In fact, these factors may elicit unwanted effects, depending on the cell type.

### Methods For The Preparation Of Antigen Specific Immunomodulatory Cells:

### Antigen(s).

The multiple sclerosis-associated antigen used in said methods for the preparation and/or generation of immunomodulatory cells may be a single antigen, plurality of antigens or cell types expressing and/or presenting said antigen or antigens. In one embodiment the antigen is selected from a group comprising of: a mixture of autoantigens derived from a patient suffering with autoimmunity, a peptide antigen, a nucleic acid, an altered peptide ligand, a recombinant protein or fragments thereof. Said multiple sclerosis-associated antigen is selected from the group comprising of Myelin Basic Protein (MBP 13-32, MBP 83-99, MBP 111- 119, MBP 146-170), Myelin Oligodendrocyte Glycoprotein (MOG 1-20, MOG 35-55) and Proteolipid Protein (PLP 139-154) and fragments, variants and mixtures thereof.

Methods for the selection, isolation, purification and preparation of such antigens are known to the person skilled in the art.

In the following, the term "immunomodulatory cells" shall be taken to mean all immunomodulatory cells prepared, expanded and/or generated by the methods of the invention described herein including both immunomodulatory cells and antigen specific immunomodulatory cells. In one embodiment said immunomodulatory cells are regulatory T-cells, in a particularly preferred embodiment said immunomodulatory cells are Foxp3+CD4+CD25+ T-reg and/or IL-10/TGFb-producing regulatory Tr1 cells.

### Further Agents.

In a preferred embodiment of the invention the methods for the preparation, expansion or generation of immunomodulatory cells are carried out in the presence of one or more agents suitable for increasing the yield of immunomodulatory cells. Preferably the step of contacting MSC with PBLs is carried out in the presence of said agents. Such agents are preferably MSC stimulating factors such as but not limited to small molecules, cytokines and or growth factors. Suitable agents include but are not limited to those selected form the group consisting of IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, LPS, G-CSF, M-CSF, GMC-SF, kit-L, VEGF, Flt-3 ligand, PDGF, FGF-2, TPO, IL-11, IGF-1, MGDF, NGF, TGF-b, HMG, thalidomide, 5-azacytidine, trichostatin-A, valproic acid, growth hormone, human chorionic gonadotropin, pituitary adenylate cyclase activating polypeptide (PACAP), serotonin, bone morphogenic protein (BMP), epidermal growth factor (EGF), transforming growth factor alpha (TGF alpha), fibroblast growth factor (FGF).

In one embodiment of the method the agent is LPS (gram negative bacterial endotoxin lipopolysaccharide). It is preferred that the LPS concentration is between 0.01 and 100 µg/ml, it is further preferred that said concentration is between 1 and 50 µg/ml e.g. about 10 µg/ml.

In an alternative embodiment said agent is either of GM-CSF and IL-4. GM-CSF and IL-4 are both cytokines. It is preferred that the concentration thereof is between 1 and 2000 IU/ml, it is further preferred that said concentration is between 500 and 1000 IU/ml.

In a further embodiment of the method both the agents IL-4 and GM-CSF are used in the method of the invention. It is preferred that the ratio of the concentration of GM-CSF to the concentration of IL-4 is between 5:1 or 1:1 and that the concentrations of each of said agents is between 1 and 2000 IU/ml, it is further preferred that said concentration is between 500 and 1000 IU/ml. Accordingly, in one embodiment this may be about 1000 IU/ml GM-CSF to 500 IU/ml IL-4.

### Immunomodulatory Cell Selection.

The disclosure provides immunoregulatory cells suitable for administration to a recipient subject. It is preferred that said immunoregulatory cells possess relative phenotypic homogeneity. Accordingly, in an optional step of the methods of the invention the immunomodulatory cells are selected from the heterogenous cell culture. The immunomodulatory cells and antigen specific immunomodulatory cells can be selected and isolated by conventional means known by a skilled person in the art. Examples of such technique include FACS and immunomagnetic cell sorting.

The present disclosure provides a method or assay for the identification of the cells. Said method comprises:
i) providing an isolated cell population;
ii) determining the expression of one, two or all markers selected from the group consisting of CD62-L, FOXP3 and CTLA4; and
iii) selecting cells positive for at least 1, 2 or 34 of said markers.

The isolated cell population of i) is preferably a cell population generated according to the method of the present invention. Methods for the detection and selection of cells according to ii) and iii) are well known in the art e.g. FACS as discussed previously. In a further embodiment in step ii) the expression of the marker CD127 is also determined and in step iii) cells negative for expression of said marker are selected.

### Cell Expansion.

In one embodiment of the method the immunomodulatory cells can be subsequently expanded in number *ex vivo* using culture techniques known in the art, or the method as disclosed herein. As an alternative treatment methodology, the immunomodulatory cells may be administered directly *in vivo.*

### Cell Storage.

The cells may be preserved by any means known in the art including but not limited to storage at room temperature in a sealed vessel, or cryopreservation.

Use Of Antigen Specific Immunomodulatory Cells Or Populations Thereof.

The disclosure also provides the use of the immunomodulatory cells, prepared, expanded and/or generated according to the methods of the invention in the treatment of multiple sclerosis, most preferably the subject from which the PBLs were obtained.

Thus, said immunomodulatory cells are used as a medicament.

In the following, the term "immunomodulatory cells" shall be taken to mean all immunomodulatory cells prepared, expanded and/or generated by the methods of the invention described herein including both expanded and non-expanded immunomodulatory cells and antigen specific immunomodulatory cells.

### Pharmaceutical Compositions.

The present invention provides pharmaceutical compositions for use in the treatment, prophylaxis, and amelioration of one or more symptoms associated with multiple sclerosis.

Thus, in another aspect, the disclosure relates to a pharmaceutical composition comprising an immunomodulatory cell and a pharmaceutical carrier. Combinations of two or more of said type of cells are included within the scope of the pharmaceutical compositions.

The pharmaceutical composition comprises a prophylactically or therapeutically effective amount of one or more prophylactic or therapeutic agents (i.e. immunomodulatory cells), and a pharmaceutical carrier. Suitable pharmaceutical carriers are known in the art and are preferably those approved by a regulatory agency of the US Federal or a state government or listed in the US Pharmacopeia, or European Pharmacopeia, or other generally recognized pharmacopeia for use in animals, and more particularly in humans. The term "carrier" refers to a diluent, adjuvant, excipient, or vehicle with which the therapeutic agent is administered. The composition, if desired, can also contain minor amounts of pH buffering agents. Examples of suitable pharmaceutical carriers are described in "Remington's Pharmaceutical Sciences" by E W Martin. Such compositions will contain a prophylactically or therapeutically effective amount of a prophylactic or therapeutic agent preferably in purified form, together with a suitable amount of carrier so as to provide the form for proper administration to the subject. The formulation should suit the mode of administration. In a preferred embodiment, the pharmaceutical compositions are sterile and in suitable form for administration to a subject, preferably an animal subject, more preferably a mammalian subject, and most preferably a human subject.

The pharmaceutical composition may be in a variety of forms. These include, for example, solid, semi-solid, and liquid dosage forms, such as lyophilized preparations, liquids solutions or suspensions, injectable and infusible solutions, etc. The preferred form depends on the intended mode of administration and therapeutic application.

### Use Of Cells In Therapy.

The administration of the immunomodulatory cell population, or the pharmaceutical composition comprising same, to the subject in need thereof can be carried out by conventional means. A composition may be prepared for systemic administration (e.g. rectally, nasally, buccally, vaginally, via an implanted reservoir or via inhalation). Alternatively, a composition may be prepared for local administration. Alternatively, a composition may be administered by the parenteral route. Alternatively, a composition may be administered by the subcutaneous, intracutaneous, intravenous, intramuscular, intra articular, intrasynovial, intrasternal, intrathecal, intralesional, intralymphatic and intracranial routes.

Said cell population may be locally administered to the subject by a method which involves transferring the cells to the desired tissue, either *in vitro* (e.g. as a graft prior to implantation or engrafting) or *in vivo,* to the animal tissue directly. The cells can be transferred to the desired tissue by any appropriate method, which generally will vary according to the tissue type. For example, cells can be transferred to a graft by bathing the graft (or infusing it) with culture medium containing the cells. Alternatively, the cells can be seeded onto the desired site within the tissue to establish a population. Cells can be transferred to sites *in vivo* using devices such as catheters, trocars, cannulae, stents (which can be seeded with the cells), etc.

The cell populations and pharmaceutical compositions can be used in a combination therapy. The combination therapy can be administered to a subject with an inflammatory disorder that is refractory to one or more anti-inflammatory agents. The combination therapy may be used in conjunction with other types of anti-inflammatory agents including, but not limited to, nonsteroidal anti-inflammatory drugs (NSAIDs), steroidal anti-inflammatory drugs, beta-agonists, anticholingeric agents, and methyl xanthines. Examples of NSAIDs include, but are not limited to, Ibuprofen, celecoxib, diclofenac, etodolac, fenoprofen, indomethacin, ketorolac, oxaprozin, nabumetone, sulindac, tolmetin, rofecoxib, naproxen, ketoprofen, etc. Such NSAIDs function by inhibiting a cyclooxgenase enzyme (e.g. COX-I and/or COX-2). Examples of steroidal anti-inflammatory drugs include, but are not limited to, glucocorticoids, dexamethasone, cortisone, hydrocortisone, prednisone, prednisolone, triamcinolone, azulfidine, and eicosanoids such as thromboxanes, and leukotrienes. Monoclonal antibodies, such as Infliximab, can also be used.

In accordance with the above, the combination therapies can be used prior to, concurrently or subsequent to the administration of such anti-inflammatory agents. Further, such anti-inflammatory agents do not encompass agents characterized herein as lymphoid tissue inducers and/or immunomodulatory agents.

### Kits.

Also disclosed are kits of use in treating a subject with the immunomodulatory cells. Said kit comprises i) an immunomodulatory cell population prepared, expanded and/or generated according to the methods of the present invention or a medicament or a pharmaceutical composition thereof and ii) a device for administering said cells such as, but not limited to, syringes, injection devices, catheters, trocars, cannulae and stents. Said kits may comprise iii) instructions for use in the treatment of a subject.

Various embodiments of the invention will be illustrated by the following examples, which illustrate but do not limit the invention described herein.

### EXAMPLES

The aim of the present experiment was to *"in vitro"* generate antigen specific Treg cells using peptides derived from proteins related to the central nervous system in the presence of allogeneic adipose derived stem cells for the treatment of multiple sclerosis and further demonstrate specificity by comparison of their suppressor activity with polyclonal Tregs or with antigen specific-Tregs generated in the presence of driven peptides.

### MATERIALS AND METHODS

Healthy donor samples: Lipoaspirates were obtained from human adipose tissue from healthy adult donors and were processed as described in DelaRosa et al. "Requirement of IFN-gamma mediated Indoleamine 2,3 dioxygenase expression in the modulation of lymphocyte proliferation by human adipose-derived stem cells." Tissue Eng Part A. (2009) 15(10):2795-2806. The hASC were used at passage 4-12. Buffy coats were provided by the National Transfusion Centre of the Comunidad Autonoma of Madrid. Peripheral blood lymphocytes were isolated from the buffy coats by density centrifugation gradient using Ficoll Paque Plus (GE Healthcare Biosciences AB, Uppsala).

### Peptide selection:

Peptides were selected based on their immunogenicity and their ability to be presented in the context of MHC II. Based on these requirements, 7 peptides were selected and instead of using one peptide per donor, a pool of the 7 peptides was used under low affinity conditions (meaning low concentration 0.1 micromolar) for the Treg induction.

Myelin Basic Protein peptides (MBP13-32, MBP 83-99, MBP 111- 119, MBP 146-170), Myelin Oligodendrocyte Glycoprotein (MOG 1-20, MOG 35-55) and Proteolipid Protein (PLP 139-154), were pooled at equal concentrations to a final stock concentration of ImM in DMSO. Pooled peptides were use in cultures at 0.1 microMolar.

The peptides used are shown in Table 1.

**Table 1**

| MYELIN BASIC PROTEIN MBP | | |
|---|---|---|
| MBP (13-32) | KYLATASTMDHARHGFLPRH | SEQ ID NO: 1 |
| MBP (83-99) | ENPVVHFFKNIVTPRTP | SEQ ID NO: 2 |
| MBP (111-119) | LSRFSWGAEGQRPGFGYGG | SEQ ID NO: 3 |
| MBP (146-170 | AQGTLSKIFKLGGRDSRSGSPMARR | SEQ ID NO: 4 |

| MYELIN OLIGODENDROCYTE GYLCOPROTEIN | | |
|---|---|---|
| MOG (1-20) | GQFRVIGPRHPIRALVGDEV | SEQ ID NO: 5 |
| MOG (35-55) | MEVGWYRPPFSRVVHLYRNGK | SEQ ID NO: 6 |

| PROTEOLIPID PROTEIN | | |
|---|---|---|
| PLP (139-154) | HCLGKWLGHPDKFVGI | SEQ ID NO: 7 |

| INFLUENZA HEMAGGLUTININ | | |
|---|---|---|
| FLUHA (306-318) | PKYVKQNTLKLAT | SEQ ID NO: 8 |

### Example 1: ASC mediated Treg generation and characterization.

Once the pool of reactive peptides to be used for the generation of specific-Tregs was selected, hASC-mediated MS-specific Treg expansions were performed.
PBMCs were co-cultured in the presence and absence of MS pooled peptides with hASCs with complete medium and IL-2 (100 UI/ml). 48 hours prior to starting the experiment ASCs were plated at 40.000 cells per well using DMEM medium supplemented with FBS. 24 hours before starting the experiments PBMCs were thawed and left under resting conditions on a non adherent well plate (to avoid adherence of monocytes) with complete RPMI . At day zero medium from ASCs was harvested and PBMCs were added to the ASC well plate at one million per ml with RPMI. Each well had 2 ml of volume. Ratio of ASC and PBMC was 40.000 per every 2 millions of PBMCs (1:50). The co-culture was performed in incubators at 37 degrees C and 5% of CO₂ in the presence and absence of 0.1 micro molar of MS pooled peptides with hASCs with RPMI complete and IL-2 (100 UI/ml). Medium was refreshed every 3 days. 15 days after co-culture cells were harvested and used for the phenotypic and functional characterization.

After 15 days of co-culture cells were harvested. A part of the cells was used to perform phenotypic characterization in order to determine the Treg phenotype. The rest of the cells were used to perform proliferation assays against CFSE stained autologous PMCS. Treg cells isolated from co-cultures with hASC and MS pooled peptides were termed TR1.Treg Cells isolated from co-cultures with hASC and no peptides were termed TR2. Treg cells isolated in the absence of hASC and in the presence of pooled peptides were TR3.Treg cells isolated in the absence of hASC and pooled peptide were termed TR4.
Two different characterization experiments were performed.

The first experiment was performed on four different co-cultures:
1. PBMC+ hASCs + IL-2 (TR2)
2. PBMC+ hASCs + peptide pool+ IL-2 (TR1)
3. PBMC + peptide pools+ IL-2 (TR3)
4. PBMC+ IL-2 (TR4)

Cultures were maintained for 15 days. After that cells from the four co-cultures were characterized by multiparametric immunofluorescence for surface markers (CD25, CD4, CD103, GITR, CD127, intracellular FOXP-3,...) and the CD4+CD25bright subset was isolated by using immunomagnetic isolation on the TR1 and TR2 co-cultured cells, which were further analysed in the following functional experiments.

The second experiment was performed on TR1 isolated Tregs.
1. PBMC+ hASCs + peptide pool+ IL-2: isolated Tregs (TR1)
Cultures were maintained for 15 days. After that cells were characterized by multiparametric immunofluorescence for surface markers (CD25, CD4, and intracellular FOXP-3) and the CD4+CD25bright subset was isolated using immunomagnetic isolation.

### Example 2: Functional evaluation of ASC-mediated myelin-specific Treg

CFSE STAINING: PBMCS from 3 donors were stained with 30µM CFSE (Sigma Aldrich) and incubated at 37°C for 15 min. The unbound CFSE was quenched by using an equal amount of FCS (Invitrogen), and, subsequently, cells were washed twice with PBS. CFSE-labeled autologous PBMCs 2 exp105cells/well were incubated in wells of flat-bottom 96-well plates at suppressor (S) ratios of 1:1, 1:4, 1:20 and 1:50. TR1 and TR2 populations were considered to mediate suppression, when they significantly inhibited proliferation of PBMCs in co-culture assays. All CFSE data were analyzed using the Cell QuestTM software.

The suppressive capacity of the myelin specific-Treg cells was tested by CFSE proliferation assay of autologous PBMCs.

The percentage of suppression was calculated by using the percentage of cells that divided more than 1 generation, by reference to a control culture of 100% proliferation or 0% suppression.

### Functional assay 1

The PBMC cells were cultured in 96-well plates together with increasing concentrations of isolated Treg cells (TR1 and TR2) reaching a final number of 200,000 cells per well. Co-cultures were left over 6 days.

To induce the proliferation of autologous lymphocytes each of the TR1 and TR2 cell populations were two using two different stimulations: polyclonal stimuli (pan T cell activation/expansion kit) and an antigen specific stimulation (using the pooled peptides that were used for the generation of Treg MS specific). Finally, cells were harvested and CFSE changes were analyzed by FACS.

### Functional assay 2

100,000 autologous PBMCs were cultured in the presence of increasing numbers of MS-specific-Tregs (TR1) over 6 days under 50 UI of IL-2 per ml plus either the pooled MS peptides or an irrelevant peptide (FLU-HA) to induce the proliferation of autologous PBMCS. Cells were then harvested and stained for CD3. CFSE changes were analyzed by FACS.

### RESULTS

### Effect of the peptide specificity on the Treg numbers and phenotype

Treg cells isolated from co-cultures with hASC and MS pooled peptides were termed TR1 Treg cells isolated from co-cultures with hASC and no peptides were termed TR2.Treg cells isolated in the absence of hASC and in the presence of pooled peptides were TR3. Treg cells isolated in the absence of hASC and pooled peptide were termed TR4.

As indicated in Figure 1 the TR1 and TR2 co-cultures presented a significantly higher percentage of Treg cells than those PBMCs induced in the absence of hASC (TR3, TR4). These data indicated that antigen driven PBMC cultures induce similar Treg percentages than non driven cultures meaning that the peptides did not affect the capacity of ASC to generate regulatory T cells.
In order to demonstrate that the antigen driven Treg (TR1) belongs to the same population as polyclonal Treg (TR2) we phenotypically analyzed the three markers characteristic of Treg cells: CD103, CD127 and FOXP3. As indicated in Figure 2 similar phenotype was found for TR1 and TR2 cells whereas non ASC induced Treg (TR3 and TR4) had lower percentage of FOXP3 and CD103. The expression of CD127 was negative in all cases as has been described for any regulatory T cell type.

### MS Specific induced Tregs:

We aimed to demonstrate the functionality of the isolated Treg cells. For this purpose CD4+CD25+ regulatory T cells from TR1 and TR2 conditions were isolated.

### Functional assay 1

For Assay 1 Autologous PBMCs 2 x 105 cells/well were incubated in flat-bottom 96-well plates at suppressor(S) ratios of 1:1, 1:4, 1:20 and 1:50. TR1 and TR2 populations were considered to mediate suppression, when they significantly inhibited proliferation of PBMCs in co-culture assays. Percentage of PBMC proliferation in the presence of increasing numbers of Treg cells was expressed by relative percentage to a 100 percent of proliferation found for PBMC alone. Results of 3 individual experiments are shown in Figures 3-8 indicating that TR1 cells were able to suppress proliferation of autologous PBMCs when MS pooled peptides were used. Comparing with a strong inductor of the proliferation like anti CD3/CD28 the suppression using MS peptides was lower, however the level of proliferation and activation reached with polyclonal stimuli is higher than the one with peptide.

TR2 polyclonal Treg, as expected didn't induce a significant decrease of the proliferation when pooled peptides were used as stimulator.

These results indicates that effectively TR1 cells, contain a higher percentage of MS specific T cells.

### Functional assay 2

For assay 2 PBMCs 1x105cells/well were incubated in flat-bottom 96-well plates at suppressor(S) ratios of 1:5, 1:11 and 1:21. Only TR1 cells were used. TR1 was considered to mediate suppression, when they significantly inhibited proliferation of PBMCs in co-culture assays. In order to study whether MS-specific Treg were able to suppress proliferation of autologous T cells only in the presence of a specific peptide, we performed the co-cultures in the presence of an irrelevant peptide. Comparing the suppressor capacity of MS specific Treg in MS induced autologous lymphocytes with MS specific Tregs in flu induced autologous lymphocytes. Figure 9 indicates percentage of proliferation in the presence of MS and flu peptide referred to the maximum proliferation reached. Results confirm that the presence of MS specific Tregs in pooled MS peptides added to PBMCS inhibit the proliferation of the autologous T cell compartment. This inhibition was significantly lower when an irrelevant peptide was used.

These results indicate that the MS-specific Tregs isolated contain a population of Treg that preferentially inhibit the autologous T cell proliferation induced in the presence of MS pooled peptides.

### CONCLUSION

Taken together, these results indicate that the method for Treg generation was able to generate a Treg cell population comprising antigen specific Treg but which retained to a significantly lesser degree a non-specific immunosuppressive capacity. In general we can also conclude that polyclonal Treg isolated from hASC/PBMC co-cultures are able to suppress the proliferation of autologous PBMCs. However the generation of MS specific Tregs preferentially inhibit autologous T cells that react against MS peptides and not other peptides (such as the irrelevant peptide FLU-HA).

### SUMMARY

Pooled MS peptides derived from proteins of the central nervous system [Myelin Basic Protein peptides (MBP13-32, MBP 83-99, MBP 111-119, MBP 146-170), Myelin Oligodendrocyte Glycoprotein (MOG 1-20, MOG 35-55) and Proteolipid Protein (PLP 139-154)] were added to the Treg induction system and MS specific Treg were generated at similar percentages as polyclonal Tregs. MS specific Treg (herein referred to as TR1) were phenotypically characterized as being CD4+CD25highFOXP3lowCD127negCD103low/neg like the polyclonal Treg cells. MS specific Treg were able to suppress the proliferation of autologous PBMCs induced with the peptide pool and low IL-2 concentration. PBMCs were also susceptible of inhibition when stimulated with anti CD3/CD28 microbeads by MS specific regulatory T cells.

Interestingly when a non-specific peptide was used, the inhibitory capacity of the MS specific Treg was reduced, therefore it can be concluded that although the cell population is "antigen-specific" in that they present a greater efficacy in the modulation of MS peptide T cell proliferation they still retain a (reduced) capacity for the modulation non-MS associated T cell response.

## Claims

1. A method for the preparation, expansion and/or generation of antigen-specific immunomodulatory cells which comprises *ex-vivo* contacting an isolated regulatory T cell population with a mesenchymal stem cell (MSC) population in the presence of one or more multiple sclerosis-associated antigens selected from the group consisting of: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6) and PLP 139-154 (SEQ ID NO: 7).

2. The method according to claim 1, which additionally comprises:
i) determining the expression of one or more markers selected from the group consisting of CD62-L, FOXP3 and CTLA4; and
ii) selecting cells positive for at least 1, 2 or 3 of said markers.

3. A method for preparing a pharmaceutical composition for use in treating multiple sclerosis, comprising the steps of:
i) providing a PBL population;
ii) contacting said PBLs with a cell population comprising mesenchymal stem cells (MSC) in the presence of one or more multiple sclerosis-associated antigens selected from the group consisting of: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6) and PLP 139-154 (SEQ ID NO: 7);
iii) isolating the immunomodulatory cell population;
iv) determining the expression of one or more markers selected from the group consisting of CD62-L, FOXP3 and CTLA4; and
v) selecting cells positive for at least 1, 2 or 3 of said markers.

4. The method according to any one of claims 1 to 3, wherein the MSC population is derived from adipose tissue.

5. The method according to any one of claims 1 to 4, wherein the immunomodulatory cells do not express CD127.

6. The method according to any one of claims 1 to 5, wherein the isolated regulatory T cell population or PBLs are contacted with a cell population comprising MSCs in the presence of a pool of multiple sclerosis-associated antigens comprising: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6) and PLP 139-154 (SEQ ID NO: 7).

## Patentansprüche

1. Verfahren zur Aufbereitung, Expansion und/oder Bildung von antigen-spezifischen immunomodulatorischen Zellen, welches umfasst das Inkontaktbringen einer isolierten regulatorischen T-Zellpopulation mit einer mesenchymalen Stammzellpopulation ex *vivo* in Gegenwart von einem oder mehreren Multiple Sklerose-assoziierten Antigenen, ausgewählt aus der Gruppe bestehend aus: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6) und PLP 139-154 (SEQ ID NO: 7).

2. Verfahren nach Anspruch 1, welches zusätzlich umfasst:
i) das Ermitteln der Expression von einem oder mehreren Markern ausgewählt aus der Gruppe bestehend aus CD62-L, FOXP3 und CTLA4; und
ii) das Auswählen von Zellen, die für mindestens 1, 2 oder 3 dieser Marker positiv sind.

3. Verfahren zum Zubereiten einer pharmazeutischen Zusammensetzung zur Verwendung in der Behandlung von Multipler Sklerose, umfassend die Schritte:
i) Bereitstellen einer PBL-Population;
ii) Inkontaktbringen der PBL mit einer Zellpopulation umfassend mesenchymale Stammzellen (MSZ) in Gegenwart von einem oder mehreren Multiple Sklerose-assoziierten Antigenen ausgewählt aus der Gruppe bestehend aus: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6) und PLP 139-154 (SEQ ID NO: 7);
iii) Isolieren der immunomodulatorischen Zellpopulation;
iv) Ermitteln der Expression von einem oder mehreren Markern ausgewählt aus der Gruppe bestehend aus CD62-L, FOXP3 und CTLA4; und
v) Auswählen von Zellen, die für mindestens 1, 2 oder 3 dieser Marker positiv sind.

4. Verfahren nach einem der Ansprüche 1 bis 3, wobei die MSZ-Population aus dem Fettgewebe stammt.

5. Verfahren nach einem der Ansprüche 1 bis 4, wobei die immunomodulatorischen Zellen CD127 nicht exprimieren.

6. Verfahren nach einem der Ansprüche 1 bis 5, wobei die isolierte regulatorische T- Zellpopulation oder PBL in Kontakt gebracht werden mit einer Zellpopulation umfassend MSZ in der Gegenwart von einem Pool von Multiple Sklerose-assoziierten Antigenen umfassend: MBP 13-32 (SEQ ID NO: 1), MBP 83-99 (SEQ ID NO: 2), MBP 111-119 (SEQ ID NO: 3), MBP 146-170 (SEQ ID NO: 4), MOG 1-20 (SEQ ID NO: 5), MOG 35-55 (SEQ ID NO: 6) und PLP 139-154 (SEQ ID NO: 7).

## Revendications

1. Procédé de préparation, d'expansion et/ou de génération de cellules immunomodulatrices à spécificité antigénique qui comprend la mise en contact *ex vivo* d'une population isolée de lymphocytes T régulatrices avec une population de cellules souches mésenchymateuses (MSC) en présence d'un ou de plusieurs antigènes associés à la sclérose en plaques sélectionnés dans le groupe constitué par : MBP 13-32 (SEQ ID N° 1), MBP 83-99 (SEQ ID N° 2), MBP 111-119 (SEQ ID N° 3), MBP 146-170 (SEQ ID N° 4), MOG 1-20 (SEQ ID N° 5), MOG 35-55 (SEQ ID N° 6) et PLP 139-154 (SEQ ID N° 7).

2. Le procédé selon la revendication 1, qui comprend en outre :
i) la détermination de l'expression d'un ou de plusieurs marqueurs sélectionnés dans le groupe constitué par CD62-L, FOXP3 et CTLA4 ; et
ii) la sélection de cellules positives pour au moins 1, 2 ou 3 desdits marqueurs.

3. Procédé de préparation d'une composition pharmaceutique destinée à être utilisée dans le traitement de la sclérose en plaques, comprenant les étapes de :
i) mise à disposition d'une population de PBL ;
ii) mise en contact desdits PBL avec une population cellulaire comprenant des cellules souches mésenchymateuses (MSC) en présence d'un ou de plusieurs antigènes associés à la sclérose en plaques sélectionnés dans le groupe constitué par : MBP 13-32 (SEQ ID N° 1), MBP 83-99 (SEQ ID N° 2), MBP 111-119 (SEQ ID N° 3), MBP 146-170 (SEQ ID N° 4), MOG 1-20 (SEQ ID N° 5), MOG 35-55 (SEQ ID N° 6) et PLP 139-154 (SEQ ID N° 7) ;
iii) isolation de la population de cellules immunomodulatrices ;
iv) détermination de l'expression d'un ou de plusieurs marqueurs sélectionnés dans le groupe constitué par CD62-L, FOXP3 et CTLA4 ; et
v) sélection de cellules positives pour au moins 1, 2 ou 3 desdits marqueurs.

4. Le procédé selon l'une quelconque des revendications 1 à 3, sachant que la population de MSC est dérivée de tissu adipeux.

5. Le procédé selon l'une quelconque des revendications 1 à 4, sachant que les cellules immunomodulatrices n'expriment pas CD127.

6. Le procédé selon l'une quelconque des revendications 1 à 5, sachant que la population isolée de lymphocytes T régulateurs ou les PBL sont mis en contact avec une population cellulaire comprenant des MSC en présence d'un ensemble d'antigènes associés à la sclérose en plaques comprenant : MBP 13-32 (SEQ ID N° 1), MBP 83-99 (SEQ ID N° 2), MBP 111-119 (SEQ ID N° 3), MBP 146-170 (SEQ ID N° 4), MOG 1-20 (SEQ ID N° 5), MOG 35-55 (SEQ ID N° 6) et PLP 139-154 (SEQ ID N° 7).
